# EUROPEAN PATENT SPECIFICATION

(11) **EP 1 781 284 B1**
(45) Date of publication and mention of the grant of the patent: **13.10.2010**
(21) Application number: 05804363.9
(22) Date of filing: 25.08.2005
(51) Int. Cl.: A61K 31/4188, A61K 38/19, A61P 35/00, A61K 49/00

(54) **USE OF THE COMBINATION COMPRISING TEMOZOLOMIDE AND TNF-ALPHA FOR TREATING GLIOBLASTOMA**
VERWENDUNG DER KOMBINATION AUS TEMOZOLOMID UND TNF-ALPHA ZUR BEHANDLUNG VON GLIOBLASTOMA
UTILISATION D'UNE COMBINAISON COMPRENANT DU TEMOZOLOMIDE ET UN TNF-ALPHA POUR LE TRAITEMENT D'UN GLIOBLASTOME

(30) Priority: 25.08.2004 US 604251 P
(43) Date of publication of application: 09.05.2007
(73) Proprietor: The University of Chicago, Chicago, IL 60637 (US); DANA-FARBER CANCER INSTITUTE, INC., Boston, MA 02115 (US)
(72) Inventor: WEICHSELBAUM, Ralph, R., Chicago, IL 60614 (US); KUFE, Donald, W., Wellesley, MA 02482 (US); YAMINI, Bakhtiar, Chicago, Illinois 60647 (US)
(74) Representative: Kremer, Simon Mark
(86) International application number: PCT/US2005/030238
(87) International publication number: WO 2006/026348

(56) References cited:
- WO-A-02/083653
- YAMINI BAKHTIAR ET AL: "Transcriptional targeting of adenovirally delivered tumor necrosis factor alpha by temozolomide in experimental glioblastoma." CANCER RESEARCH. 15 SEP 2004, vol. 64, no. 18, 15 September 2004 (2004-09-15), pages 6381-6384, XP009059976 ISSN: 0008-5472
- ROEHN T A ET AL: "CCNU-dependent potentiation of TRAIL/Apo2L-induced apoptosis in human glioma cells is p53-independent but may involve enhanced cytochrome c release" ONCOGENE, BASINGSTOKE, HANTS, GB, vol. 20, no. 31, 12 July 2001 (2001-07-12), pages 4128-4137, XP002336509 ISSN: 0950-9232
- SAITO RYUTA ET AL: "Convection-enhanced delivery of tumor necrosis factor-related apoptosis-inducing ligand with systemic administration of temozolomide prolongs survival in an intracranial glioblastoma xenograft model." CANCER RESEARCH. 1 OCT 2004, vol. 64, no. 19, 1 October 2004 (2004-10-01), pages 6858-6862, XP009059977 ISSN: 0008-5472

## Description

### INTRODUCTION

Despite aggressive treatment of malignant glioma, there has been little improvement over the past 30 years in the survival of patients with malignant gliomas. Radiation therapy (IR) remains the mainstay of post-surgical management. Recently, the concurrent use of the oral alkylating agent temozolomide (TMZ) with IR has been shown to modestly increase prognosis in patients who have undergone complete surgical resection (Stupp, R. et al. (2005) N Engl J Med 352:987-996). Promising investigational targeted therapies (Castro, M.G. et al. (2003) Pharmacol Ther 98:71-108), such as targeted toxins, monoclonal antibodies or immune mediated approaches, have yet to make a significant clinical impact. A number of factors account for the poor response of malignant brain tumors to therapy, including the intrinsic resistance of glioma cells to DNA damage-induced cytotoxicity (Taghian, A. et al. (1995) Int J Radiat Oncol Biol Phys 32:99-104) (Johnstone, R.W. et al. (2002) Cell 108:153-164) and the normal tissue toxicity produced by currently employed therapeutic agents. Investigation of combination treatment strategies that activate complementary cytotoxic pathways is an important aspect of developing anti-cancer treatments that overcome resistance to treatment and improve patient prognosis (Vivo, C. et al. (2003) J Biol Chem 278:25461-25467).

TMZ is a monofunctional alkylating agent with a favorable toxicity profile commonly used in the treatment of malignant glioma. Although the combined use of TMZ and IR is now a preferred regimen for the treatment of both newsy diagnosed and recurrent glioblastoma, the prognosis for people with malignant glioma remains dismal.

Therefor, there exists a need in the art for improved methods, pharmaceutical, and therapeutic combinations for treating people with malignant glioma.

### SUMMARY OF THE INVENTION

The present invention is as defined in the accompanying claims. Described herein is a method of synergistically inhibiting growth of a glioma cell comprising contacting the cell with temozolomide and TNFα.

Described herein is a method of synergistically inhibiting growth of a glioma in a human cancer patient comprising the use of temozolomide and a vehicle comprising or expressing TNFα, wherein the vehicle is to be administered directly to the glioma.

Described herein is a method of synergistically inhibiting growth of a glioma in a human cancer patient comprising the use of temozolomide, a vehicle comprising or expressing TNFα, and radiation, wherein the vehicle and irradiation are to be administered directly to the glioma.

Also provided are a pharmaceutical combination comprising temozolomide and a vehicle comprising or expressing TNFα, and a therapeutic combination comprising temozolomide, a vehicle comprising or expressing TNFα, and radiation.

### BRIEF DESCRIPTION OF THE DRAWINGS

Fig. 1 shows the amount of TNFα produced by U87 malignant glioma cells transfected with adenovirus expressing TNFα under the control of an Egr-1 promoter *in vitro* (Fig. 1A) and *in vivo* (Fig 1B) in response to exposure to TMZ
Fig. 2A shows the percent cell viability, as measured by the tryptan blue dye exclusion method, of U87 malignant glioma cells subjected to different treatments; Fig. 2B shows the optical densities (490 nm), obtained using the MTS colorimetric assay, for U87 malignant glioma cell populations exposed to different treatments.
Fig. 3A shows the fractional tumor volume (V/V₀) of hindlimb glioma tumors as a function of time post exposure to different treatments; Fig. 3B shows the percent survival (in days) for populations of mice with hindlimb glioma tumors exposed to different treatments as a function of time.
Fig. 4 shows apoptosis, as measured by TUNEL positive U87 glioma cells /10⁻⁶mm² as a function of treatment.
Fig. 5 shows Kaplan-Meier survival curves of nude mouse intracranial xenografts.

### DETAINED DESCRIPTION OF THE INVENTION

The present disclosure provides methods for synergistically inhibiting or reducing the growth of malignant glioma cells using pharmaceutical or therapeutic combinations. The method includes use of a pharmaceutical combination of temozolomide (TMZ) and tumor necrosis factor- alpha (TNFα), or a therapeutic combination comprising TMZ, TNFα, and radiation therapy (IR). Thus, the present invention provides a therapeutic approach to treating malignant glioblastoma.

Before any embodiments of the invention are explained in detail, it is to be understood that the invention is not limited to the details of the invention set forth in the following description or illustrated in the appended figures. The invention is capable of other embodiments and of being practiced or of being carried out in various ways. Also, it is to be understood that the phraseology and terminology used herein is for the purpose of description and should not be regarded as limiting.

As used herein, the term "synergistically inhibits" means that the total inhibitory effect of the agents administered is greater than the sum of the individual inhibitory effects of the agents.

The term "contacting" is used herein interchangeably with the following: combined with, treating, added to, mixed with, passed over, incubated with, etc.

As used herein, "radiation" or "radiation therapy" refers to all known and appropriate forms of radiant energy (e.g., alpha, beta, gamma and x-rays as well as protons) that are commonly used in cancer treatment and delivered by any known method of delivery, for example, from an external source (beam), radiation from a radiation source implanted proximal to the tumor, radiation from a radionuclide attached to monoclonal antibodies or a compound that targets the cancer, radiation in a gamma knife, 3D conformal radiation, and radiation in steriotactic radiosurgery.

The therapeutic or pharmaceutical combinations described herein are meant to refer to a combination therapy or treatment by any administration route in which two or more therapeutic agents, including modalities such are radiation, are administered to cells, to a patient or to a subject. For combination treatment with more than one active agent, where the active agents are in separate formulations or modalities, the active agents can be administered concurrently, or they each can be administered at separately staggered times. The agents may be administered simultaneously or sequentially, as long as they are given in a manner sufficient to allow both agents to achieve effective concentrations in the cell or body. The agents may be administered by different routes, e.g., one agent may be administered intravenously while a second agent is administered intramuscularly, intravenously or orally.

In time-sequential administration, one agent may directly follow administration of the other or the agents may be give episodically, i.e., one can be given at one time followed by the other at a later time, e.g., within 2-3 days, or one can be given daily while another is given episodically, e.g., every 2-3 days. Suitable time-sequential administration in accordance with the present invention is detailed in the Examples below.

The pharmaceutical compositions used in the pharmaceutical or therapeutic combinations of this invention may be administered orally, parenterally, by intratumoral injection, by inhalation spray, topically, rectally, nasally, buccally, vaginally or via an implanted reservoir. Oral administration or administration by injection is most common. The pharmaceutical compositions of this invention may contain any conventional non-toxic pharmaceutically-acceptable carriers, adjuvants or vehicles.

The dosage amount of the compositions in accordance with the present invention for treating a patient is an amount sufficient to inhibit or reduce growth of a glioma cell or tumor. Specific dosage and treatment regimens for any particular patient will depend upon a variety of factors, including the activity of the specific compound employed, the age, body weight, general health status, sex, diet, time of administration, rate of excretion, drug combination, the severity and course of the infection, the patient's disposition to the infection and the judgment of the treating physician. Thus, the number of variables in regard to an individual treatment regimen is large, and a considerable range of doses is expected.

In an illustrated embodiment, the invention provides a method of synergistically inhibiting or reducing the growth of glioma cells by contacting the cells with a combination TMZ and TNFα, i.e., the cells are treated or contacted with both agents. Temozolomide is an imidazotetrazine derivative having the structure:

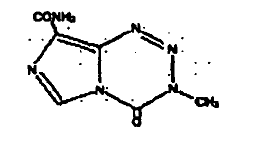

Temozolomide is commonly and conveniently administered orally in capsule form. However, it should be appreciated that TMZ could be also be administered by any other suitable means, e.g., intraperitoneally (IP), as shown in the Examples below.

In the methods of the invention, glioma cells are contacted with TNFα by contacting the glioma cells with a vehicle comprising or expressing TNFα. Such vehicles may include, for example, a liposome or nanoparticle comprising the TNFα polypeptide, or an expression vector, such as a viral vector comprising a polynucleotide encoding the TNFα polypeptide operably linked to a promoter functional in the host cell. Preferably, the promoter is an inducible promoter responsive to TMZ and/or IR, such as a promoter comprising the CArG elements of the Egr-1 promoter. Suitably, the viral vector is an adenovirus vector, preferably a replication defective adenovirus vector.

In the Examples, Ad.Egr-TNF was injected into the glioma, followed by intraperitoneal administration of TMZ, or IR and TMZ. As one of ordinary skill in the art will appreciate, the order in which various components of the pharmaceutical combination or therapeutic combination are administered is not critical. Some routine optimization may be involved to insure that TMZ and/or IR are present at a level sufficient to induce expression of TNFα, or that TMZ is present at a time and concentration effective to inhibit NFκB, or to act synergistically with TNFα to increase apoptosis or cytotoxicity.

TNFα is a prototypical death ligand and induces cytotoxicity via the extrinsic apoptosis cascade following binding to its cell surface receptor. However, TNFα-induced cytotoxicity is abrogated by preferential activation of the pro-survival transcription factor, nuclear factor-κB (NF-κB) (Karin, M. and Lin, A. (2002) Nat Immunol 3:221-227), which confers resistance to the cells. Activation of NF-κB has also been shown to mediate resistance to other genotoxic stressors such as IR (Wang, C.Y. et al. (1996) Science 274:784-787), and inhibition of TNFα- or IR-induced NF-κB has been shown to potentiate the cytotoxicity of these agents (Beg, A.A. and Baltimore, D. (1996) Science 274-782-784) (Van Antwerp, D.J. et al. (1996) Science 274:787-789) (Yamagishi, N. et al. (1997) Int J Radiat Biol 72:157-162) (Miyakoshi, J. and Yagi, K. (2000) Br J Cancer 82:28-33).

Briefly, the NF-κH family consists of five structurally related proteins the most abundant form of which consists of the heterodimer of p50 (NF-κB1) and p65 (RelA). In unstimulated cells, NF-κB is sequestered in the cytosol bound to inhibitor of-κB protein (IκBα). Following TNFα stimulation, IκBα is phosphorylated and degraded releasing the NF-κB subunits which translocate into the nucleus, bind to DNA and activate transcription. Phosphorylation of IκBα occurs following activation of the IκBα kinase (IκK) complex. In addition to IκBα degradation, NF-κB transcriptional activity has also been shown to be regulated by post-translational modification of the p65 subunit.

As described in the Examples below, the effects of combinations of TMZ and Ad.Egr-TNF, and TMZ, IR and Ad.Egr-TNF were evaluated in mouse hindlimb and intracranial malignant glioma xenografts, two models of human malignant glioma. Therapy was shown to be significantly more effective than the current standard anti-glioma regimen of TMZ and IR, by several different criteria, including extended survival, reduced tumor volume, enhanced apoptosis, and enhanced cytotoxicity. TMZ-mediated inhibition of TNFα- and IR-induced NF-κB activation is responsible, at least in part, for the enhanced results obtained using these combinations. Furthermore, enhanced interaction between TNFα and TMZ leads to the accumulation of reactive oxygen species (ROS), resulting in delayed c-Jun N-terminal kinase (JNK) activation that mediates tumor cell apoptosis.

The following Examples are intended to be purely illustrative.

### EXAMPLES

**Example 1. Reagents and cells.** TMZ was supplied by Schering Corporation (Kenilworth, New Jersey, USA) and was dissolved in DMSO with the final concentration not exceeding 0.1 % (v/v). DMSO and human TNFα were obtained from Sigma (St. Louis, Missouri, USA). N-acetylcysiein (NAC) was obtained from Roxane Laboratories, Inc. (Columbus, Ohio, USA). Annexin V-FITC apoptosis detection kit II was manufactured by BD Pharmingen (San Jose, California, USA). Hydroethidine (HE) was purchased from Molecular Probes, Invitrogen Detection Technologies (Eugene, Oregon, USA). SP600125 was purchased from (EMD Bioscience, San Diego, California, USA). Human glioblastoma cell lines: U87 MG, T98MG, U251, pancreatic cancer cells: Panc1, MLkPaCa-2 and BxPC-3 and esophageal cancer cells: Seg-1 were purchased from American Type Culture Collection and cultured in DMEM supplemented with 10% FBS (Intergen Co., Purchase, New York, USA), penicillin (100IU/mL), and streptomycin (100µg/mL) (Invitrogen Life Technologies, Carlsbad, California, USA) at 37°C and 5% CO₂.

**Example 2. Plasmids and virus.** The expression vectors pRC-CMV-p65, pRC-CMV and green fluorescent protein (GFP) have been previously described (Tang, F. et al. (2002) Mol Cell Biol 22:8571-8579). The NF-κB luciferase reporter construct, Ig-κB-Luc, containing three repeats of the immunoglobulin κ-light chain enhancer xB site and the Egr-1 promoter luciferase construct, pE425 GL3, have also been previously described (Park, J.O. et al. (2002) J Clin Invest 110:403-410) (Kanno, T. et al. (1995) J Biol Chem 270:11745-11748). The replication incompetent adenoviral vector, Ad.Egr-TNF, was described in U.S. Provisional Application No. 60/604,251 (Yamini, B., et al. (2004) Cancer Res 64:6381-6384). Ad.Egr-TNF (GenVec Inc., Gaithersburg, Maryland) was stored at -80°C and diluted in formulation buffer to the appropriate concentration. Temozolomide (Schering Corporation, Kenilworth, New Jersey) was dissolved in DMSO with the final concentration not exceeding 0.1% (v/v).

**Example 3. TNFα induction** ***in vitro.*** 10⁶ U87 cells were plated and incubated overnight. The cells were then infected with Ad.Egr-TNF at a multiplicity of infection (MOI) of 100 for 3 h at 37°C. After incubation, 3.8 mL of complete media with or without TMZ was added. Conditioned media were harvested at 48 h after treatment and human TNFα production was quantified using a Quantikine ELISA kit (R&D System Inc., Minneapolis, Minnesota).

**Example 4. TNFα induction** ***in vivo.*** U87 cells (5 x 10⁶) in 100 µL DMEM were injected subcutaneously (sc) into the right hindlimb of nude mice. When tumors reached an average size of 200 mm³ (length x width x thickness/2), the tumor-bearing mice were randomized into 4 groups: 1. Untreated Control (UTC); 2. Ad.Egr-TNF alone; 3. TMZ alone; 4. Ad.Egr-TNF and TMZ. Ad.Egr-TNF was injected intratumorally (IT) at a dose of 2 x 10⁸ particle units (pu) each day. Two doses of TMZ were given: 2.5mg/kg/day and 5mg/kg/day by intraperitoneal (IP) injection 3 h after vector. Four consecutive daily IT and IP injections were given, control animals received IT and IP serum free medium (SFM). Animals were euthanized on day 2 and 4 (i.e., 48 h and 96 h after treatment initiation), tumors harvested, snap-frozen in liquid nitrogen and homogenized in RIPA buffer (150 mM NaCl, 10 mM Tris at pH 7.5, 5 mM EDTA at pH 7.5, 100 mM PMSF, 1 µg/mL leupeptin, and 2 µg/mL aprotinin). Protein was isolated and concentration measured using Protein Assay reagent (Bio-Rad Laboratories, Hercules, California). TNFα levels in the supernatants were measured as described above.

**Example 5. U87 cell viability studies.** Trypan Blue dye exclusion method was employed. 10⁴ U87 cells were plated and incubated at 37°C overnight. Subsequently, the cells were contacted with media containing TNFα (10 ng/mL) and/or TMZ (100 µM), incubated for 3 h, and washed. At 24 h, 48 h, and 72 h following exposure to agent, the cells were trypsinized and the viable cell number/well determined using a hemocytometer. Cell viability at 72 h was verified using the MTS colorimetric assay, per the manufacturer's protocol (Cell Titer 96 Aqueous, One Solution cell proliferation assay; Promega Corporation, Madison, Wisconsin, USA). Optical density was read at 490 nm using an ELISA microplate reader after 1.5h, at 37°C. All of the studies were performed in triplicate.

### Example 6. Xenograft Studies.

*Hindlimb Studies*: U87 hindlimb xenografts were established as described above in Example 4. In one study, mice were randomized into four groups as described in Example 4 and treatment initiated (day 0). Ad.Egr-TNF (2 x 10⁸ pu) was injected IT twice a week for 4 total injections, and 5 mg/kg TMZ was given IP 3 h after each vector injection for a total of 20 mg/kg. The dose of TMZ used was approximately 0.2 LD₁₀ and was chosen to have modest anti-tumor effect but to not be curative based on previous studies (Friedman, H.S. et al. (1995) Cancer Res 55(13):2853-2857) and data from our lab showing LD₅₀ for IP TMZ to be approximately 500 mg/kg. Tumor volume was measured every 2-3 days. Fractional tumor volume (V/V₀ where V₀ = volume on day 0) was calculated and plotted.

In a second study, tumor-bearing mice were randomized into eight treatment groups: untreated control (UTC); intratumoral (IT) Ad.Egr-TNF alone; intraperitoneal (IP) TMZ alone; IR alone; Ad.Egr-TNF and TMZ; Ad.Egr-TNF and IR; TMZ and IR; and Ad.Egr-TNF, TMZ and IR. Ad.Egr-TNF was administered IT at a dose of 2 x 10⁸ pu/ 10 µL twice a week for 2 weeks, IP TMZ was given 3 h after vector at 5 mg/kg to a total of 20mg/kg. Animals were placed in Lucite chambers and given 5 Gy IR to the tumor 1 h before TMZ (on days when both TMZ and IR were administered), every 2-3 days to a total of 30 Gy. For all controls, animals were injected IT or IP with serum free medium (SFM) and animals were also placed in chambers without IR. Xenografts were measured twice a week using calipers, tumor volume was calculated, and fractional tumor volumes (*V*/*V*₀ where *V₀* = volume on day 0) were plotted.

*Intracranial Studies:* In two separate experiments, 5 x 10⁵ U87 cells were inoculated into the right caudate nucleus on day 0 using a screw guide technique (Lal, S. et al. (2000) J Neurosurg 92:326-333). In the first experiment, *mice* were randomized into four groups as described above in Example 4. On day 5, a single intracranial (IC) injection of 5 x 10⁸ pu Ad.Egr-TNF in 5 µl volume was made directly into the tumor using the screw guide technique. TMZ (5 mg/kg) was given IP 3 h after IC vector inoculation. Three additional IP TMZ injections were administered on consecutive days for a total dose of 20 mg/kg. Control animals received SFM IT and IP.

In a second experiment, mice were randomized into eight groups (untreated control (UTC); intratumoral (IT) Ad.Egr-TNF alone; intraperitoneal (IP) TMZ alone; IR alone; Ad.Egr-TNF and TMZ; Ad.Egr-TNF and IR; TMZ and IR; and Ad.Egr-TNF, TMZ and IR). A single dose of 5 x 10⁵ U87 cells was inoculated into the right caudate nucleus of each mouse on day 0 using a screw guide technique (Lal, S. et al. (2000) J Neurosurg 92:326-333). On day 5, 5 x 10⁸ pu Ad.Egr-TNF in 5 µL volume was injected once via the screw guide directly into the tumor. Beginning two hours after vector injection, 5Gy IR was delivered to the tumor area and repeated every 2- 3 days to a total of 30Gy. TMZ (5 mg/kg) was given IP 3 h after vector inoculation and like doses were given daily for the next two days for a total dose of 15 mg/kg. Control animals received SFM IT and IP and were also placed in Lucite chambers. Daily assessment of animal appearance was made. Mice were followed until death or sacrificed when moribund. Mouse brains were harvested following intracardiac perfusion and fixed with 10% neutral buffered formalin. For TUNNEL evaluation (see below) animals were sacrificed on day 7 following treatment (n = 3 per group).

### Example 7. Flow cytometric analysis of apoptosis.

*Fractional DNA content*: U87 cells (10⁵) were plated overnight at 37°C with 5% CO₂. The cells were then treated with TNFα (10ng/mL) and /or TMZ (100µM). At 72 h the cells were washed in PBS and fixed, in ice-cold 70% (v/v) ethanol. The cells were washed twice and incubated in RNase (1mg/mL) for 30 m at 37°C, then incubated in propidium iodide (PI) solution (100ug/mL) for 30 m at 4°C. Flow cytometric analysis was performed on a FACSort instrument (Becton Dickinson Immunocytometry Systems, San Jose, California), and the data were analyzed using the CellQuest software (Becton Dickinson).

*Annexin V binding* (van Engeland, M. et al. (1998) Cytometry 31(1):1-9): At 72 h cells were washed in PBS and incubated in the dark for 15 m with binding buffer containing 5 µl of Annexin V-FITC and 5 µl of PI (Annexin V-FITC apoptosis detection kit II). The data was analyzed by Flowjo analysis software (Tree Star Inc., Ashland, Oregon).

**Example 8. Histological Analysis.** Paraffin embedded brains were sectioned (8 µm), stained with hematoxylin and eosin and analyzed in a blinded fashion.

Terminal deoxynucleotidyl transferase-mediated dUTP-biotin nick end-labeling (TUNEL) assay was performed in accordance with the manufacturer's instructions (Chemicon) and analyzed blindly at 400x magnification by use of a computer-aided light microscope with reconstruction software (Neurolucida, Microbrightfield, Vt). Number of TUNNEL positive cells per 10⁻⁶ mm² was documented.

**Example 9. Luciferase assay.** U87 cells (5 x 10³) were plated overnight and subsequently co-transfected with Ig-κB-Luc (or pE425 GL3) and the *Renilla reniformis* expression vector, pRL-TK, to normalize transfection efficiency, at a ratio of 10:1 using SuperFectin transfection kit (Qiagen, Valencia, California, USA). Twenty-four hours after transfection, the cells were pretreated with TMZ (100µM) for 16 h, then treated with TNFα (10ng/mL). Five hours later NF-κB (or Egr-1) and *Renilla* luciferase activity were measured with the Dual-Luciferase reporter assay system (Promega Corp., Madison, Wisconsin, USA). Relative luciferase was calculated as the ratio of firefly luminescence / *Renilla* luminescence for each sample.

**Example 10. Preparation of nuclear extracts.** Confluent cultures of U87 cells were grown in complete medium and then left untreated or treated with 10 ng/mL TNFα for 20 m and 1 h +/-16 h pre-treatment with 100µM TMZ (or 0.1% DMSO control vehicle). Cells were then washed with 10 mL ice-cold PBS, scraped from the dish, and pelleted by centrifugation at 1000 rpm for 5 m at 4°C. Cell pellets were resuspended in 400 µl of ice-cold buffer A (10 mM HEPES, pH 7.9; 10 mM KCl; 0.1 mM EDTA; 1 mM DTT; 0.5 mM phenylmethysulfonylfluoride [PMSF]; 1 µg/mL leupeptin; 5 µg/mL aprotinin) and allowed to swell on ice for 15 m. Following the addition of 25 µl of 10% NP-40, the suspension was vortexed for 10 s and centrifuged at 14,500 rpm for 1 m at 4°C. Nuclei were resuspended in 50 µl of ice-cold buffer B (20 mM HEPES, pH 7.9; 0.4 NaCl; I mM EDTA; 1 mM DTT; 1 mM PMSF; 25% glycerol; 1 µg/mL leupeptin; 5 µg/mL aprotinin) and incubated on ice for 15 m. The nuclear suspension was then centrifuged at 14,500 rpm for 5 m at 4°C and the supernatant containing the nuclear proteins was transferred to a clean tube. Protein concentrations for each sample were determined by the Bradford method (Bio-Rad, Richmond, California, USA) and were adjusted to 2 µg/µl by the addition of buffer B.

**Example 11. Electrophoretic mobility shift assay.** Assays were performed using the Promega gel shift assay system. NF-κB consensus oligonucleotide (oligo) (5'AGTTGAGGGGACTITCCCAGGC3') (SEQ ID NO:1) was end labeled with [γ-³²P] ATP using T4 polynucleotide kinase and incubated for 10 m at 37°C. The reaction was stopped by the addition of 1 µl of 0.5 M EDTA. Binding reactions contained the following: 5 µl nuclear extract (10 µg protein), 2 µl distilled deionized water, and 2 µl of 5 × gel shift binding buffer (20% glycerol; 5 mM MgCl2; 2.5 mM EDTA; 2.5 mM DTT; 250 mM NaCl; 50 mM Tris-HCl, pH 7.5; 0.25 mg/mL poly(dI-dC)poly(dI-dC). The reaction mixture was incubated at room temperature for 10 m, and then 1 µl (0.035 pmol) of ³²P-Iabeled NF-κB oligo was added. After an additional 20 m, the reaction was stopped by adding 1 µl of 10× gel loading buffer (250 mM Tris-HCl, pH 7.5; 0.2% bromophenol blue; 40% glycerol). 10 µL were loaded onto a 5% non-denaturing polyacrylamide gel and run in 0.5 x TBE (45 mM Tris-HCl, 45 mM boric acid, 1 mM EDTA) for 1 h. The gel was dried under a vacuum at 80°C for 1 h and exposed to photographic film at -70°C. For competitor reactions, 10 ng of TNFα treated U87 nuclear extract was incubated for 30 m with 50-fold excess of unlabeled NF-κB consensus sequence oligo (specific competitor) or unlabeled AP-1 consensus sequence oligo (non-specific competitor). Supershift studies were performed by 30 m pre-incubation of nuclear extracts from TNFα treated cells with antibody against p65 or p50 (Active Motif, Carlsbad, California, USA).

**Example 12. Western blot analysis.** 20µg of whole U87 cell (or nuclear) lysate was subjected to sodium dodecyl sulfate (SDS)-polyacrylamide gel electrophoresis (PAGE). Following electro-transfer, Immobilon-P membranes (Millipore Corp. Burlington, Massachusetts, USA.) were probed with primary polyclonal antibody against IκBα, phospho-Ser32-IxBa, SAPK/JNK, phospho-Thr183/Tyr185-SAPK/JNK, p65, phospho-Ser536-p65 (Cell Signaling Technology Inc. Beverly, Maryland, USA) diluted 1:1000 overnight at 4°C. Anti-rabbit IgG HRP-linked secondary antibody (Cell Signaling Technology) was diluted 1:1000 in blocking buffer and applied for 1 h at room temperature. Immunoreactive bands were detected by SuperSignal enhanced chemiluminescence (ECL) (Pierce, Rockford, Illinois, USA) and exposed to Kodak X-Omat film.

**Example 13. Annexin V binding.** Cells were either un-transfected or co-transfected with pRC-CMV-p65 or pRC-CMV in the presence of a GFP plasmid at a ratio of 4:1. Under these conditions, cells expressing GFP also expressed the co-transfected plasmid (Tang, F. et al. (2002) Mol Cell Biol 22:8571-8579). Cells were then left untreated or treated as described in the figure legends. At 72 h cells were washed in PBS and incubated in the dark for 15 m with binding buffer containing 5 µl of Annexin V-FITC and 5µl of Propidium iodide (PI). In transfected cells, annexin V binding was assessed in GFP positive cells. Data was analyzed by Flowjo analysis software (Tree Star Inc., Ashland, Oregon, USA) as described in U.S. Provisional Application No. 60/604,251 (Yamini, B. et al. (2004) Cancer Res 64:6381-6384).

**Example 14. Protein kinase assay.** IκK was immunoprecipitated from treated U87 cell extracts with anti-IKKβ antibody. (Santa Cruz Biotechnology, Santa Cruz, California, USA). The kinase activity of the immune complex was assayed at 30°C for 30 to 60 m in 30 µl of kinase buffer (Mercurio, F. et al. (1997) Science 278:860-866*)* in the presence of 10 µM ATP- 10 µCi [γ-³²P]ATP Dupont NEN with (GST)-IκBα (1-54) protein (purified on glutathione-agarose beads as described (DiDonato, J.A. et al. (1997) Nature 388:548-554)) as a substrate. The reaction was terminated with 4x Laemmli sample buffer and proteins resolved by SDS-12% PAGE. Kinase activity was quantified using a Phosphoimager and Equal protein loading determined by immunoblotting with anti-IκKβ antibody (Upstate USA, Charlottesville, Virginia, USA). The antibody-antigen complexes were visualized by the ECL detection system (Amersham, England).

**Example 15. Measurement of superoxide (O₂) production.** U87 cells were plated at a density of 10⁶ cells in flat-bottom 6-well tissue culture plates, incubated overnight and treated as indicated in the figure legend. Cells were then washed, resuspended in PBS and 1 µL of 10mM hydroethidine (HE) per mL cell suspension (10µM final concentration) was added and incubated for 5 m at 37°C. Cells were harvested and analyzed on a flow cytometer (FACSort; BD Biosciences) with excitation at 488nm and emission collected using a 620-670nm absorbance long-pass filter. Data was analyzed by Flowjo software.

**Example 16. Statistical analysis.** Results are expressed as mean value ± SEM. Statistical significance was taken as *P<* 0.05 using a one-tailed student *t-*test. Analysis of variance (ANOVA) was also employed. Kaplan-Meier survival curves were plotted for the intracranial experiment and analyzed by the Log-rank method.

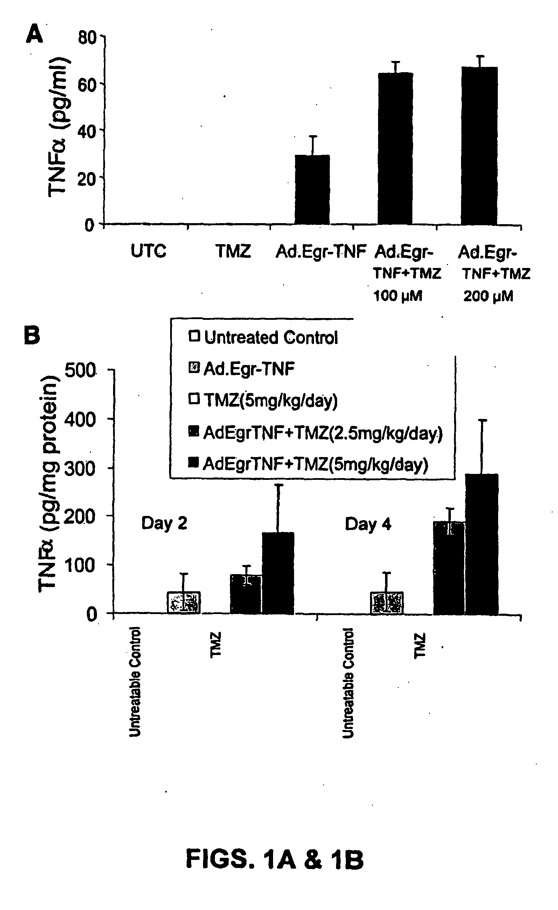

**Example 17. TMZ induced TNFα reduced U87MG cell viability.** TMZ was found to induce expression of TNFα from U87 cells infected with Ad.Egr-TNF. In *in vitro* studies, TNFα was detected in untreated control cells or in cells treated with TMZ alone. Following Ad.Egr-TNF infection, 100 µM TMZ induced a 2.3-fold increase in TNFα expression compared to cells infected with vector alone (Fig.1*A*). Hindlimb xenografts were used to evaluate *in vivo* induction. No TNF was detected in the untreated animals or in animals treated with TMZ alone (Fig. 1*B*). However, following combination treatment with Ad.Egr-TNF/TMZ, 287 ± 111 pg TNFα/mg protein was detected at 96 h, 6.4 times more TNFα found in glioma cells of animals treated with Ad.Egr-TNF alone (*n*= 3 animals per group, *P* = 0.02) (Fig. 1*B*).

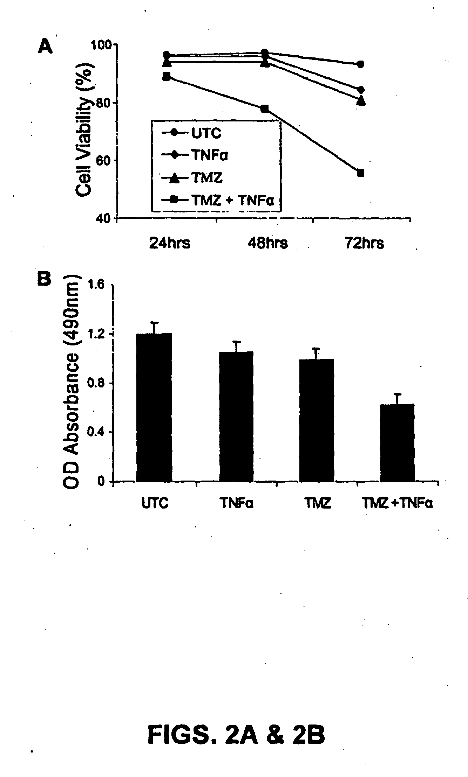

The cytotoxic effect of TNFα and TMZ on glioma cell viability was evaluated *in-vitro.* Minimal effects on U87 cell viability was observed in U87 cells treated with either 10 ng/mL TNFα or 100 µM TMZ alone. However, combination treatment led to a significant reduction in cell viability, the magnitude of which was greater than the sum of the reductions of either treatment alone (Fig. 2*A* and *B*). That a synergistic interaction between TNFα and TMZ exists is supported by analysis of variance (ANOVA) assessment (*P* = 0.0016)

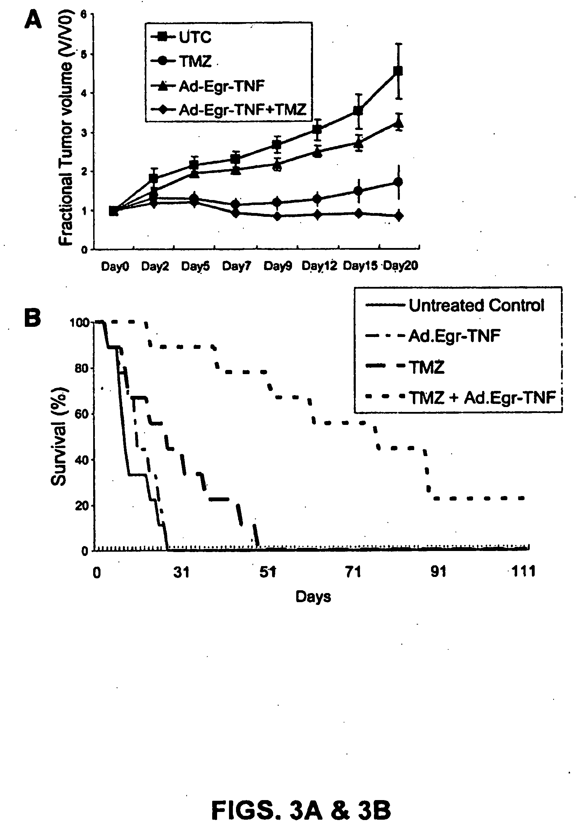

**Example 18. Combination of TNFα and TMZ exhibited anti-tumor efficacy.** The anti-tumor efficacy of TNFα and TMZ was evaluated in hindlimb xenografts. Treatment of tumors with Ad.Egr-TNF alone did not significantly affect growth kinetics relative to growth kinetics of untreated control animals. Fractional tumor volume of animals treated with Ad.Egr-TNF and TMZ was significantly smaller compared to the fractional tumor volume of animals treated with TMZ alone (*P* < 0.02 at day 20) (Fig. 3A). In intracranial xenograft experiments, nude mouse survival was recorded following treatment. Treatment with 20mg/kg TMZ alone prolonged median survival over that of untreated control animals and animals treated with Ad.Egr-TNF only (28 days vs. 18 and 21 days, respectively). No mice lived past day 48. However, median survival of animals following combination treatment with Ad.Egr-TNF/ TMZ was significantly increased to 76 days (*P* < 0.001 by log-rank compared to TMZ alone) (Fig. 3*B*). The animals in all the treatment groups appeared healthy. Histological assessment of intracranial sections showed decreased cell density in the combined treatment group with minimal oligodendroglial toxicity and, most significantly, there was no increase in tumor necrosis when compared to either treatment alone (data not shown).

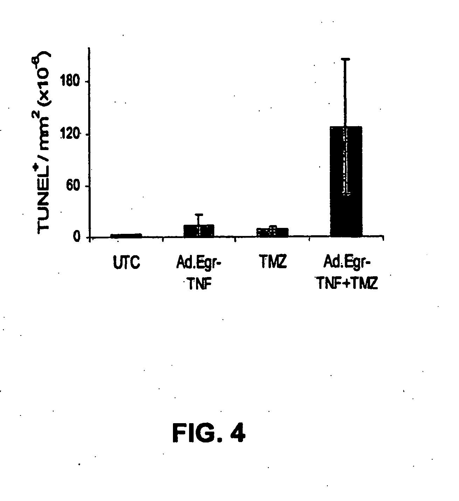

**Example 19. TMZ and TNFα act synergistically to enhance apoptosis.** Flow cytometric (FACS) analysis of U87 cells was used to assess the fractional DNA content following treatment. As expected, TNFα alone had minimal effect on U87 cell apoptosis and TMZ alone led to an increase in the percentage of cells in G2/M phase. However, treatment with both TNFα and TMZ lead to a significant increase in the sub-G1 (hypodiploid/apoptotic) peak at 72 h, compared to either treatment alone (*P* <0.05). Annexin V staining of U87 cells confirmed results obtained by FACS. Combination treatment led to a 9-fold and 3.3-fold increase in annexin V positive cells compared to those treated with only TNFα or TMZ, respectively, at 72 h. The interaction between TNFα and TMZ leading to apoptosis was determined to be synergistic as assessed by ANOVA (*P* < 0.05). To determine whether the synergistic effect of TNFα and TMZ on apoptosis observed *in vitro* also occurs *in vivo,* intracranial tumor sections, specifically, sections taken during the early stage of treatment (day 7), were evaluated using TUNEL. Tumors treated with Ad.Egr-TNF/TMZ combination had significantly more TUNEL positive cells than those treated with either TMZ or Ad.Egr-TNF alone (110 ± 77 vs. 14± 12 and 13±13 TUNEL⁺ cells 10⁻⁶ mm² respectively, *P* < 0.05) (Fig. 4).

The synergy between TNFα and TMZ provides enhanced efficacy in inhibiting growth of glioma cells over the use of TMZ alone. Although TMZ has relatively mild side effects, the maximal dose that can be safely administered is limited by hematological toxicity. The therapeutic index of TMZ can be greatly enhanced when TMZ used in a combination treatment strategy with virally delivered TNFα.

These results are unexpected in light of previous reports. Eggermont et al. reported that an observed synergistic interaction between high dose TNFα and an alkylating agent in isolated limb perfusion studies was due to increased tumor necrosis, possibly resulting from increased vascular permeability leading to an increase in intratumoral drug concentration (Eggermont, A.M. et al. (2003) Lancet Oncol 4(7):429-437). A similar pattern of tumor necrosis has also been observed when radiotherapy is combined with Ad.Egr-TNF in a flank glioma model (Staba, M.J. et al. (1998) Gene Ther 5(3):293-300). In contrast, the combination of TMZ and TNF produced no histologically detectable necrosis, and instead caused a significant increase in tumor cell apoptosis both *in vitro* and *in vivo,* whereas neither TNFα alone nor TMZ alone causes significant apoptosis in glioma cells. Considered together, these data strongly suggest that there is a direct interaction between TNFα and TMZ in glioma cells that enhances apoptosis resulting in the therapeutic benefit reported in our experiments.

A therapeutic increase in tumor cell apoptosis has been speculated to be a desirable goal of novel glioma therapies (Raza, S.M. et al. (2002) Neurosurgery 51(1):2-12; discussion 12-3) particularly because tumor necrosis has been associated with a significantly worse prognosis in GBM patients (Lacroix, M. et al. (2001) J Neurosurg 95(2):190-198). However, further studies are necessary to determine the mechanism involved in the induction of apoptosis and to evaluate whether treatment-induced apoptosis yields a greater therapeutic ratio in malignant glioma than therapeutically induced necrosis.

Mortality from malignant glioma is related primarily to recurrent disease, which is almost universally local (non-metastatic) in nature (20). For this reason, a regionally activated treatment strategy is especially suitable for treating such tumors.

**Example 20. Temozolomide inhibits TNFα-induced NF-κB transcription in glioma cells.** Because TNFα is known to induce a pro-survival transcription factor NF-κB, the activation of which mediates resistance to other genotoxic stressors, the effect of TMZ TNFα-induced NF-κB was evaluated, as was the question of whether any such effect contributes to the apoptotic/cytotoxic interaction between TMZ and TNFα. In an NF-κB-responsive luciferase reporter assay, TMZ pre-treatment was shown to cause dose dependent inhibition of TNFα-induced NF-κB transcriptional activity in U87 glioma cells (*P* = 0.002 TNFα + TMZ 100 µM compared to TNFα alone). TMZ has a similar effect on TNFα-induced NF-κB activity in tested human gliome cell linesT98 and U251. In contrast, TMZ. activated TNFα-induced NF-κB transcriptional activity in human pancreatic and esophageal cancer cell lines (Panel, MIAPaCa-2, BxPC-3 and Seg-1). These results suggest that the inhibition of TNFα-induced NF-κB activity by TMZ may be selective for human glioma cells compared to other cancer cell types.

**Example 21. Temozolomide suppresses TNFα-induced NF-κB nuclear translocation, nuclear translocation, and activation.** The results of experiments undertaken to further characterize the effect of TMZ on TNFα-induced NF-κB indicated that TMZ inhibits TNFα-induced NF-κB transcriptional activity in part by inhibiting NF-κB nuclear translocation. Additionally, NF-κB induced to translocate to the nucleus following TNFα stimulation in glioma cells was shown to contain the p65 subunit as a major component. TMZ does not directly affect TNFα-induced NF-κB DNA binding. Additionally, TMZ inhibits TNFα-induced degradation of IκBα, an upstream regulator of NF-κB. The data from the experiments performed as described above in the previous Examples showed that TNFα treatment caused complete degradation of IκBα at 15 m and that, although TMZ pretreatment had little effect on overall IκBα protein levels, it reduced TNFα-induced degradation. Furthermore, increasing concentrations of TMZ resulted in greater inhibition of TNFα-induced IκBα degradation at 15 m. These results suggest that TMZ inhibits TNFα-induced NF-κB activity at least in part by inhibiting TNFα-induced IκBα degradation. TMZ pretreatment also reduced TNFα-induced phosphorylation of IκBα, a reaction catalyzed by IκKI, by 50% at 5 m. In addition, TMZ inhibits alters TNFα-induced p65 phosphorylation, which has the effect of reducing TNFα-induced NF-κB nuclear translocation. Inhibition of TNFα-induced p65 phosphorylation is overcome by overexpression of p65.

**Example 22. Temozolomide induces prolonged JNK activation that contributes to tumor cell apoptosis.** Sustained JNK activation has been shown to mediate both TNFα- and DNA damage-induced apoptosis in the setting of reduced NF-κB activation (Tang, G. et al. (2001) Nature 414:313-317) (Benhar, M. et al. (2001) Mol Cell Biol 21:6913-6926). Therefore, experiments were undertaken to evaluate whether TMZ and TNFα affect JNK phosphorylation (activation) in glioma cells. As shown previously, glioma cells have baseline activation of JNK (Antonyak, M.A. et al. (2002) Oncogene 21:5038-5046). Initial experiments initially demonstrated that although TNFα transiently increased JNK activity, treatment with TMZ and TNFα led to a biphasic increase in JNK phosphorylation, with the delayed phase occurring approximately 20 h following treatment. TMZ treatment alone resulted in a progressive and delayed activation of JNK. Because JNK activation has been previously shown to occur as a result of caspase activation (Cardone, M.H. et al. (1997) Cell 90:315-323), cells were pretreated with the general caspase inhibitor, zVAD-fmk prior to assessing JNK activation. The results showed that even though zVAD completely reversed the cytotoxicity induced by combined treatment with TMZ and TNFα, it did not inhibit the delayed JNK activation induced by this combination. In fact, JNK activation was shown to be even greater following zVAD pretreatment.

Whether prolonged JNK activation is necessary for apoptosis U87 cells was assessed using annexin V binding following treatment with TNFα and TMZ in the presence of the specific JNK inhibitor SP600125. Pretreatment with SP600125 inhibited both transient and delayed JNK activation following stimulation with TMZ and TNFα, and SP600125 reverses the apoptosis induced by treatment with combination TMZ and TNFα (*P* < 0.01 TNFα + TMZ + SP600125 compared to TNFα + TMZ). The effect of SP600125 on cell death was confirmed using an MTS assay of U87 cells at 72 h following treatment. Taken together, these data suggest that JNK activation is necessary for apoptosis following TNFα and TMZ treatment but that the JNK activation, as seen with TMZ treatment alone, is not sufficient to induce apoptosis.

**Example 23. Reactive oxygen species (ROS) mediate delayed JNK phosphorylation and induction of apoptosis following combination TMZ and TNFα treatment.** ROS have been shown to mediate the sustained component of TNFα-induced JNK activation in cells that have a defect in NF-κB activation (Sakon, S. et al. (2003) Embo J 22:3898-3909). Whether TMZ and TNFα treatment results in accumulation of ROS in U87 cells was evaluated using the cell permeable dye hydroethidine (HE), which is oxidized by superoxide radicals (O₂) to the fluorescent ethidium. Combination treatment with TMZ and TNFα led to a progressive increase in the accumulation of O₂^{·-} over 24 h as evidenced by an increase in the intensity of ethidium, and this increase in O₂⁻ was significantly inhibited by pretreatment with the antioxidant NAC (*P* < 0.05 TNFα + TMZ + NAC compared to TNFα + TMZ). Additionally, pretreatment of U87 cells with NAC reduces the delayed JNK activation induced by combination TMZ and TNFα treatment by 1.7-fold without affecting transient JNK activation.

Whether ROS play a role in apoptosis induced by combination TNFα and TMZ treatment was evaluated. Pretreatment of U87 cells with NAC had minimal effects on cell death. However, NAC significantly reversed the apoptosis induced by combination TMZ and TNFα treatment (*P* < 0.01 TNFα + TMZ + NAC compared to TNFα + TMZ). Next, to assess a direct link between p65 and ROS, HE oxidation was evaluated in cells co-transfected with HA-p65 (or empty vector) and a GFP expression vector. The results indicate that p65 over-expression significantly reduced HE oxidation following TNFα and TMZ treatment compared to control (*P* < 0.05).

The combination of TNFα and TMZ increase O₂⁻ species, and inhibition of ROS results in inhibition of delayed JNK activation and apoptosis. JNK activation is downstream of ROS accumulation, which is in contrast to previous reports (Ventura, J.J. et al. (2004) Genes Dev 18:2905-2915). The results indicate that p65 inhibits the ROS accumulation induced by TNFα and TMZ treatment while having no significant effect on basal ROS production.

**Example 24. Combination TMZ, IR and Ad.Egr-TNF suppress hindlimb glioma regrowth.** IR plays a major role in the management of malignant glioma (Walker, M.D. et al. (1980) N Engl J Med 303:1323-1329) and the combined use of IR and Ad.Egr-TNF causes tumor regression by a mechanism involving both direct tumor toxicity and an indirect anti-vascular effect (Weichselbaum, R.R. et al. (2002) Lancet Oncol 3:665-671). Whether the addition of IR could significantly enhance the anti-tumor effect of TMZ and TNFα was evaluated. A complete disappearance of palpable tumor in hindlimb glioma xenografts in nude mice (10/10) treated with TMZ, IR and Ad.Egr-TNF was appreciated at 30 days following treatment 10/10 animals. In contrast, 1/10 animals treated with TMZ alone, IR alone, IR and TMZ, or TMZ/Ad.Egr-TNF groups (*P* < 0.00001 TMZ+ IR + Ad.Egr-TNF compared to Ad.Egr-TNF + TMZ). These data demonstrate a potent anti-tumor interaction *in vivo*.

**Example 25. TMZ suppresses IR- and TNFα-induced NF-κB activity and nuclear translocation** ***in vivo.*** Because IR-induced NF-κB activation has been shown to mediate radiation resistance in tumor cells, the inhibitory effect of TMZ on TNFα-induced NF-κB *in vivo* was evaluated. Co-treatment of glioma cells with TNFα and IR increase NF-κH transcriptional activity and nuclear translocation, and these TNFα and IR effects are inhibited by TMZ in a dose-dependent matter.

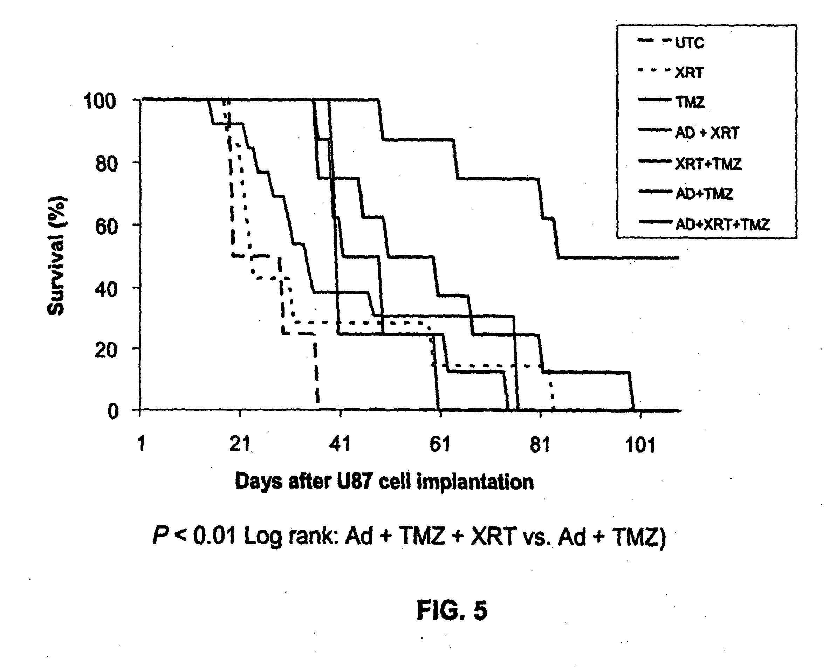

**Example 26. Triple therapy with Ad.Egr-TNF, TMZ and IR leads to an increase in animal survival in an intracranial glioma xenograft model.** The results obtained by treating mice having a hindlimb glioma xenograft with TMZ, IR and Ad.Egr-TNF were confirmed using an intracranial glioma xenograft model. Survival, of mice treated with Ad.Egr-TNF, IR and TMZ alone and in combination was evaluated. Animals treated with Ad.Egr-TNF, IR and TMZ were found to have a significant increase in median survival compared to all other treatment groups, and specifically compared to the standard anti-glioma regimen of IR and TMZ. 50% of the animals treated with TMZ, IR and Ad.Egr-TNF were still alive and appeared healthy 100 days post tumor inoculation, compared to 0% of animals in all other treatment groups (*P* < 0.01 Ad.Egr-TNF + IR + TMZ vs. Ad.Egr-TNF + TMZ) (Fig. 5).

The use of TMZ with concomitant IR has become a standard initial strategy for the management of patients with malignant glioma. Nevertheless, prognosis for these patients remains dismal. The heterogeneous nature of malignant glioma suggests that a multimodal therapeutic strategy that incorporates conventional chemo/radiotherapy with newer experimental approaches will be needed to achieve better outcomes (Guha, A. and Mukherjee, J. (2004) Curr Opin Neurol 17:655-662). One potentially promising approach for the management of cancer has been to target death ligands, such as TNFα, to trigger apoptosis in tumor cells. This is an attractive approach as death ligands can directly activate the apoptotic cascade in part through different mechanisms than those activated by DNA damaging agents (Ashkenazi, A. (2002) Nat Rev Cancer 2:420-430). Although glioma cells have been shown to be resistant to cytotoxicity induced by the TNFα superfamily (Sakuma, S. et al. (1993) Neurooncol 15:197-208) (Knight, M.J. et al. (2004) Mol Carcinog 39:173-182), treatment in combination with chemotherapeutic agents has been shown to sensitize cells to death ligand induced cytotoxicity (Vivo, C. et al. (2003) J Biol Chem 278:25461-25467) (Yamini, B. et al. (2004) Cancer Res 64:6381-6384) (Duan, L. et al. (2001) J Neurooncol 52:23-36) (Saito, R. et al. (2004) Cancer Res 64:6858-6862).

Intracranially induced TNFα (delivered by Ad.Egr-TNF) in combination with TMZ and IR significantly increases the survival of animals bearing an intracranial glioma xenograft compared to survival of animals achieved with the current standard anti-glioma treatment regimen of IR and TMZ. Importantly, the animals treated with triple therapy appeared healthy with no early treatment-related deaths.

Activation of the transcription factor NF-κB mediates resistance to TNFα, IR and chemotherapy (Wang, C.Y. et al. (1996) Science 274:784-787) (Beg, A.A. and Baltimore, D. (1996) Science 274:782-784) (Wang, C.Y. et al. (1999) Nat Med 5:412-417). Inhibition of NF-κB activation has been shown to sensitize tumor cells to TNFα- and IR-induced apoptosis (Van Antwerp, D.J. et al. (1996) Science 274:787-789) (Yamagishi, N. et al. (1997) Int J Radiat Biol 72:157-162). Although TNFα and IR increase NF-κB activity, diverse chemotherapeutic agents have been shown to both increase and reduce NF-κB activity (Das, K.C. and White, C.W. (1997) J Biol Chem 272:14914-14920) (Campbell, K.J. et al. (2004) Mol Cell 13:853-865) (Chuang, S.E. et al. (2002) Biochem Pharmacol 63:1709-1716). This study provides the fist evidence that TMZ strongly inhibits the transcriptional activity of TNFα-induced NF-κB. When used alone, TMZ slightly increases the transcriptional activity of IVF-κB in glioma cells. Therefore, the observation that TNFα-induced NF-κB activity is completely inhibited by TMZ is quite unexpected. Other chemotherapeutic agents not in general clinical use were previously reported to inhibit TNFκ-induced NF-κB activity (Ichikawa, H. et al. (2005) J Immunol 174:7383-7392). Much emphasis and research is currently focused on the development of clinically useful inhibitors of the NF-κB activation pathway (Karin, M. et al. (2004) Nat Rev Drug Discov 3:17-26) (Aggarwal, B.B. (2004) Cancer Cell 6:203-208). TMZ is a commonly used DNA alkylator with a favorable toxicity profile and its inhibition of TNFα- and IR-induced NF-κB potentially represents a novel and clinically useful mechanism by which death ligands and conventional DNA damaging agents can be combined in the management of malignant glioma. The results disclosed herein indicate that TMZ suppresses TNFα-induced NF-κB activity in several glioma cell lines (U251, T98 and U87 cells), but not in pancreatic or esophageal cancer cell lines, which suggests that TMZ-mediated inhibition of NF-κB activation may be specific for glioma cells.

## Claims

1. The use of temozolomide in the preparation of a medicament for the treatment of malignant glioma, by inhibiting growth of a glioma cell, said treatment being a combination treatment with TNFα.

2. The use of TNFα in the preparation of a medicament for the treatment of malignant glioma, by inhibiting growth of a glioma cell, said treatment being a combination treatment with temozolomide.

3. The use of claim 1 or claim 2, wherein the.TNFa is provided by a vehicle comprising or expressing TNFα.

4. The use of any of claims 1-3, wherein the glioma cell is within a glioma of a human cancer patient.

5. The use of any one of claims 1-4. wherein the temozolomide inhibits TNFα-induced transcription, nuclear translocation, or activation of NFκB.

6. The use of any of claims 1-5. wherein temozolomide increases c-Jun N-terminal kinase activity.

7. The use of any of claims 1-6, wherein temozolomide inhibits p65 phosphorylation.

8. The use of claim 1 wherein the treatment uses a vehicle comprising or expressing TNFα, wherein the vehicle is administered directly to the glioma in a human cancer patient.

9. The use of claim 2 wherein the treatment uses a vehicle comprising or expressing TNFα wherein the vehicle is administered directly to the glioma in a human cancer patient.

10. The use of any of claims 1-9, wherein the treatment is correlated with increased glioma cell cytotoxicity or apoptosis relative to that of glioma cells treated with only TNFα or only temozolomide.

11. The use of any of claims 1-10, wherein the treatment is not correlated with increased necrosis of the glioma cells.

12. The use of any one of claims 1-11, the treatment further comprising the use of ionizing irradiation to inhibit growth of the glioma cell.

13. The use of any one of claims 1-12, wherein the glioma cell is a malignant glioma cell.

14. The use of claim 8 or claim 9, said treatment further comprising administering radiation directly to the glioma.

15. The use of any of claims 3-14, wherein the TNFα is supplied by a vehicle comprising or expressing TNFα, wherein said vehicle composes an engineered adenovirus comprising a nucleotide sequence encoding TNFα operably linked to a chemoinducible or radioinducible promoter.

16. The use of claim 15, wherein the promoter comprises the CArG elements of the Egr-1 promoter.

17. The use of any of claims 3-16, wherein the TNFα is supplied by a vehicle comprising or expressing TNFα, said vehicle being a genetic construct.

18. The use of any one of claims 4-17, wherein the TNFα is supplied by a vehicle comprising or expressing TNFα, and vehicle is administered intratumorally.

19. The use of any of claims 1-18, wherein the temozolomide induces expression of TNFα.

20. The use of any of claims 4-19, wherein the treatment reduces the volume of the glioma.

21. The use of any one of claims 4-20, wherein the treatment increases the survival of the patient relative to the survival of a control or control population receiving temozolomide alone.

22. The use of any of claims 4-21, wherein the treatment increases survival of the patient relative to a control or control population receiving a subcombination of temozolomide, a vehicle comprising or expressing TNFα, and radiation.

23. The use of any of claims 4-22, wherein the glioma is physically associated with the brain, spinal cord, or optic nerve.

24. A pharmaceutical combination comprising temozolomide and a vehicle comprising or expressing TNFα.

25. The combination of claim 24, further comprising a radionuclide.

26. The combination of claim 24 or claim 25 for use in a method of treatment of malignant glioma.

## Patentansprüche

1. Verwendung von Temozolomid zur Herstellung eines Medikaments zur Behandlung von malignen Gliomen durch Hemmung des Wachstums einer Gliomzelle, wobei die Behandlung eine Kombinationsbehandlung mit TNFα darstellt.

2. Verwendung von TNFα zur Herstellung eines Medikaments zur Behandlung von malignen Gliomen durch Hemmung des Wachstums einer Gliomzelle, wobei die Behandlung eine Kombinationsbehandlung mit Temozolomid darstellt.

3. Verwendung nach Anspruch 1 oder Anspruch 2, worin der TNFα mittels eines Vehikels bereitgestellt ist, das TNFα umfasst oder exprimiert.

4. Verwendung nach einem der Ansprüche 1 bis 3, worin die Gliomzelle in einem Gliom eines menschlichen Krebspatienten liegt.

5. Verwendung nach einem der Ansprüche 1 bis 4, worin das Temozolomid TNFαinduzierte Transkription, Kerntranslokation oder Aktivierung von NFκB hemmt.

6. Verwendung nach einem der Ansprüche 1 bis 5, worin Temozolomid die N-terminale c-Jun-Kinaseaktivität steigert.

7. Verwendung nach einem der Ansprüche 1 bis 6, worin Temozolomid p65-Phosphorylierung hemmt.

8. Verwendung nach Anspruch 1, worin bei der Behandlung ein Vehikel eingesetzt wird, das TNFα umfasst oder exprimiert, wobei das Vehikel direkt an das Gliom eines menschlichen Krebspatienten verabreicht wird.

9. Verwendung nach Anspruch 2, worin bei der Behandlung ein Vehikel eingesetzt wird, das TNFα umfasst oder exprimiert, worin das Vehikel direkt an das Gliom in einem menschlichen Krebspatienten verabreicht wird.

10. Verwendung nach einem der Ansprüche 1 bis 9, worin die Behandlung mit erhöhter Gliomzellzytotoxizität oder -apoptose im Vergleich zu jener von Gliomzellen, die mit TNFα alleine oder Temozolomid alleine behandelt werden, einhergeht.

11. Verwendung nach einem der Ansprüche 1 bis 10, worin die Behandlung nicht mit erhöhter Nekrose der Gliomzellen einhergeht.

12. Verwendung nach einem der Ansprüche 1 bis 11, wobei die Behandlung weiters die Verwendung von ionisierender Strahlung zur Hemmung des Wachstums der Gliomzelle umfasst.

13. Verwendung nach einem der Ansprüche 1 bis 12, worin die Gliomzelle eine maligne Gliomzelle ist.

14. Verwendung nach Anspruch 8 oder Anspruch 9, wobei die Behandlung weiters die direkte Bestrahlung des Glioms umfasst.

15. Verwendung nach einem der Ansprüche 3 bis 14, wobei der TNFα mittels eines Vehikels bereitgestellt ist, das TNFα umfasst oder exprimiert, worin das Vehikel ein gentechnisch verändertes Adenovirus umfasst, das eine für TNFα kodierende Nucleotidsequenz umfasst, die operabel an einen chemisch induzierbaren oder durch Strahlung induzierbaren Promotor gebunden ist.

16. Verwendung nach Anspruch 15, worin der Promotor die CArG-Elemente des Egr-1-Promotors umfasst.

17. Verwendung nach einem der Ansprüche 3 bis 16, worin der TNFα mittels eines Vehikels bereitgestellt ist, das TNFα umfasst oder exprimiert, wobei das Vehikel ein genetisches Konstrukt ist.

18. Verwendung nach einem der Ansprüche 4 bis 17, worin der TNFα mittels eines Vehikels bereitgestellt ist, das TNFα umfasst oder exprimiert, und das Vehikel intratumoral verabreicht wird.

19. Verwendung nach einem der Ansprüche 1 bis 18, worin das Temozolomid die Expression von TNFα induziert.

20. Verwendung nach einem der Ansprüche 4 bis 19, worin die Behandlung das Volumen des Glioms verringert.

21. Verwendung nach einem der Ansprüche 4 bis 20, worin die Behandlung das Überleben des Patienten im Vergleich zum Überleben einer Kontrolle oder Kontrollpopulation, die Temozolomid alleine erhält, steigert.

22. Verwendung nach einem der Ansprüche 4 bis 21, worin die Behandlung das Überleben des Patienten im Vergleich zu einer Kontrolle oder Kontrollpopulation, die eine Subkombination aus Temozolomid, einem TNFα umfassenden oder exprimierenden Vehikel und Strahlung erhält, steigert.

23. Verwendung nach einem der Ansprüche 4 bis 22, worin das Gliom physisch mit dem Gehirn, dem Rückenmark oder dem Sehnerv verbunden ist.

24. Pharmazeutische Kombination, die Temozolomid und ein TNFα umfassendes oder exprimierendes Vehikel umfasst.

25. Kombination nach Anspruch 24, die weiters ein Radionuclid umfasst.

26. Kombination nach Anspruch 24 oder Anspruch 25 zur Verwendung in einem Verfahren zur Behandlung eines malignen Glioms.

## Revendications

1. Utilisation de témozolomide dans la préparation d'un médicament pour le traitement d'un gliome malin, en inhibant la croissance d'une cellule de gliome, ledit traitement étant un traitement de combinaison avec TNF_{α}.

2. Utilisation de TNF_{α} dans la préparation d'un médicament pour le traitement d'un gliome malin, en inhibant la croissance d'une cellule de gliome, ledit traitement étant un traitement de combinaison avec le témozolomide.

3. Utilisation selon la revendication 1 ou la revendication 2, où le TNF_{α} est réalisé par un véhicule comprenant ou exprimant TNF_{α}.

4. Utilisation selon l'une quelconque des revendications 1-3, où la cellule de gliome est dans un gliome d'un patient cancéreux humain.

5. Utilisation selon l'une quelconque des revendications 1-4, où le témozolomide inhibe la transcription induite par TNF_{α}, translocation nucléaire ou activation de NFκB.

6. Utilisation selon l'une quelconque des revendications 1 à 5, où le témozolomide augmente l'activité kinase c-Jun N-terminale.

7. Utilisation selon l'une quelconque des revendications 1 à 6, où le témozolomide inhibe la phosphorylation p65.

8. Utilisation selon la revendication 1, où le traitement utilise un véhicule comprenant ou exprimant TNF_{α}, où le véhicule est administré directement au gliome dans un patient cancéreux humain.

9. Utilisation selon la revendication 2, où le traitement utilise un véhicule comprenant ou exprimant TNF_{α}, où le véhicule est administré directement au gliome dans un patient cancéreux humain.

10. Utilisation selon l'une quelconque des revendications 1-9, où le traitement est mis en corrélation avec une cytotoxicité de cellule de gliome ou apoptose augmentée relativement à celle des cellules de gliome traitées seulement avec TNF_{α} ou seulement témozolomide.

11. Utilisation selon l'une quelconque des revendications 1 à 10, où le traitement n'est pas mis en corrélation avec une nécrose accrue des cellules de gliome.

12. Utilisation selon l'une quelconque des revendications 1 à 11, le traitement comprenant en outre l'utilisation d'un rayonnement ionisant pour inhiber la croissance des cellules de gliome.

13. Utilisation selon l'une quelconque des revendications 1 à 12, où la cellule de gliome est une cellule de gliome maligne.

14. Utilisation selon la revendication 8 ou la revendication 9, ledit traitement comprenant en outre l'admiistration du rayonnement directement au gliome.

15. Utilisation selon l'une quelconque des revendications 3 à 14, où le TNF_{α} est fourni par un véhicule comprenant ou exprimant TNF_{α}, où ledit véhicule comprend un adénovirus manipulé comprenant une séquence de nucléotides codant pour TNF_{α} fonctionnellement liée à un promoteur chimio-inductible ou radio-inductible.

16. Utilisation selon la revendication 15, où le promoteur comprend les éléments CArG du promoteur Egr-1.

17. Utilisation selon l'une quelconque des revendications 3-16, où le TNF_{α} est fourni par un véhicule comprenant ou exprimant TNF_{α}, ledit véhicule étant un produit de synthèse génétique.

18. Utilisation selon l'une quelconque des revendications 4 à 17, où le TNF_{α} est fourni par un véhicule comprenant ou exprimant TNF_{α}, et le véhicule est administré de manière intratumorale.

19. Utilisation selon l'une quelconque des revendications 1-18, où le témozolomide induit l'expression de TNF_{α}.

20. Utilisation selon l'une quelconque des revendications 4 à 19, où le traitement réduit le volume du gliome.

21. Utilisation selon l'une quelconque des revendications 4 à 20, où le traitement augmente la survie du patient relativement à la survie d'un contrôle ou d'une population de contrôle recevant le témozolomide seul.

22. Utilisation selon l'une quelconque des revendications 4 à 21, où le traitement augmente la survie du patient relativement à un contrôle ou une population de contrôle recevant une sous-combinaison de témozolomide, un véhicule comprenant ou exprimant TNF_{α} et un rayonnement.

23. Utilisation selon l'une quelconque des revendications 4-22, où le gliome est associé physiquement au cerveau, à la moelle épinière ou au nerf optique.

24. Combinaison pharmaceutique comprenant du témozolomide et un véhicule comprenant ou exprimant TNF_{α}.

25. Combinaison selon la revendication 24, comprenant en outre un radionucléide.

26. Combinaison de la revendication 24 ou de la revendication 25 pour utilisation dans une méthode de traitement d'un gliome malin.
